# EUROPEAN PATENT SPECIFICATION

(11) **EP 1 408 123 B1**
(45) Date of publication and mention of the grant of the patent: **09.09.2009**
(21) Application number: 03256302.5
(22) Date of filing: 07.10.2003
(51) Int. Cl.: C12R 1/19, C12P 13/08, C07K 14/215, C12N 15/90

(54) **A microorganism in which the fadR gene is knocked out and a process for producing L-threonine using the microorganism**
Mikroorganismus mit einem "knock-out" fadR Gen und dessen Verwendung in einem Verfahren zur Herstellung von L-Threonin
Microorganisme dans lequel le gène fadR a été inactivé et procédé de production de L-thréonine utilisant ledit microorganisme

(30) Priority: 11.10.2002 KR 2002062103
(43) Date of publication of application: 14.04.2004
(73) Proprietor: CJ Cheiljedang Corporation, Jung-gu, Seoul 100-749 (KR)
(72) Inventor: Park, Young Hoon, Seongnam-si, Gyeonggi-do, 463-703 (KR); Lee, Jin Ho, Ichcon-si, Gyeonggi-do, 467-711 (KR); Lee, Byoung Choon, Seongbuk-gu, Seoul, 136-075 (KR); Kim, Dae Cheol, Paldal-gu, Suwon-si, Gyeonggi-do 442-746 (KR); Cho, Jae Young, Yongin-si, Gyeonggi-do, 449-843 (KR)
(74) Representative: Lee, Nicholas John

(56) References cited:
- EP-A- 1 347 057
- WO-A-03/038106
- MALOY S R ET AL: "GENETIC REGULATION OF THE GLYOXYLATE SHUNT IN ESCHERICHIA-COLI" JOURNAL OF BACTERIOLOGY, vol. 149, no. 1, 1982, pages 173-180, XP009022005 ISSN: 0021-9193
- RAMAN NARAYAN ET AL: "Characterization of the fatty acid-responsive transcription factor FadR: Biochemical and genetic analyses of the native conformation and functional domains" JOURNAL OF BIOLOGICAL CHEMISTRY, vol. 272, no. 49, 5 December 1997 (1997-12-05), pages 30645-30650, XP002265259 ISSN: 0021-9258
- DIRUSSO C C: "NUCLEOTIDE SEQUENCE OF THE FAD-R GENE A MULTIFUNCTIONAL REGULATOR OF FATTY ACID METABOLISM IN ESCHERICHIA-COLI" NUCLEIC ACIDS RESEARCH, vol. 16, no. 16, 1988, pages 7995-8010, XP001156329 ISSN: 0305-1048

## Description

The present invention relates to an L-threonine-producing microorganism and a process for producing L-threonine using the microorganism. More particularly, it is directed to a mutated microorganism with the ability to produce L-threonine in high yields, in which the *fadR* gene of the microorganism has been "knocked out" so that the expression of the aceBAK operon is not partially repressed during the L-threonine biosynthesis. This may be achieved by using a site-specific homologous recombination system, such as *cre* gene and loxp site.

L-threonine is an essential amino acid and is widely used as a feed and food additive, and also as a pharmaceutical and raw material for synthesizing some drugs. It has been produced by fermentation with artificial mutants of the genus *Escherichia, Coryneform bacteria, Seratia* and *Providencia.* For example, Japanese Patent Publication No. S56-10037 discloses a method for producing L-threonine using a strain belonging to the genus *Escherichia* which has a nutritional requirement for diaminopimeric acid and methionine, and has the resistance to the feedback inhibition by threonine of the biosynthetic system of threonine. Japanese Patent Application Laid-open No. S58-224684 discloses a method for producing L-threonine using a strain belonging to the genus *Brevibacterium* which is resistant to S-(2-aminoethyl)-L-cysteine and α-amino-β-hydroxy valeric acid and has a nutritional requirement for L-isoleucine and L-lysine. Korean Patent Application Laid-open No. 87-8022 discloses a method for producing L-threonine using a methionine-requiring, α-amino-β-hydroxy valeric acid-resistant strain belonging to the genus *Escherichia* which has an additional resistance to at least one substance selected from the group consisting of rifampicin, lysine, methionine, aspartic acid, and homoserine, and has a reduced ability to decompose L-threonine. Japanese Patent Application Laid-open No. H2-219582 discloses a method for producing L-threonine using a strain belonging to the genus *Providencia* which is resistant to α-amino-β-hydroxy valeric acid, L-ethionine, thioisoleucine, oxythiamine, and sulphaguanidine, and has a nutritional requirement for L-leucine and also a leaky requirement for L-isoleucine.

However, the above known methods have the disadvantages that they fail to afford a high production of L-threonine or require costly nutritional requirements such as diaminopimiric acid and isoleucine. In other words, the use of diaminopimeric acid-requiring strains in the production of L-threonine includes an additional fermentation of diaminopimeric acid and thus may increase cost. Where a strain having a nutritional requirement for isoleucine is used for the production of L-threonine, costly isoleucine must be added to fermentation media, which increases cost.

In an attempt to overcome these disadvantages, the present inventors developed an L-threonine-producing strain of *Escherichia coli* which is resistant to L-methionine, L-threonine and L-lysine analogues and α-aminobutyric acid, and has a nutritional requirement for methionine and a leaky requirement for isoleucine. They successfully produced L-threonine by fermentation with the strain at higher yields than with prior strains. The strain and a method for producing L-threonine using said strain are disclosed in Korean Patent Publication No. 92-8365.

Maloy & Nunn, Journal of Bacteriology, 149(I):173-180, 1982 discloses an *E.coli* strain (RS3040) having a transposon inserted in and disrupting the *fadR* gene (*fadR*::Tn10).

The present inventors have now developed a strain capable of producing L-threonine in high yields by knocking out the *fadR* gene on its chromosome and have successfully obtained a remarkably improved yield of L-threonine by fermentation with said strain.

In a first aspect, the present invention provides an L-threonine-producing *E. coli* which is resistant to α-aminobutyric acid, D,L-ethionine, α-amino-β-hydroxy valeric acid, and S-(2-aminoethyl)-L-cystein and in which the *fadR* gene has been knocked out.

In a second aspect, the present invention provides a method for producing an *E. coli* of the first aspect, which comprises (a) introducing a knockout cassette of the *fadR* gene into an L-threonine-produeing *E*. *coli* which is resistant to α-aminobutyric acid, D,L-ethionine, α-amino- β-hydroxy valeric acid, and S-(2-aminoethyl)-L-cystein, to cause homologous recombination to occur between the cassette and the *fadR* gene of the *E. coli,* and (b) selecting a mutated *E. coli* in which the *fadR* gene has been rendered knocked-out.

In a third aspect, the present invention provides a process for producing L-threonine which comprises culturing the *E.coli* of the first aspect and isolating L-threonine from the culture. The process may use fermentation.

Preferred features of each aspect of the invention are set out in the dependent claims herein.

The present inventors have continued to develop a strain with the ability to produce L-threonine at more increased yields, and have focused on the *fadR* gene. The product of this gene is a regulatory factor for the expression of the fatty acid decomposition-associated operon (fad operon) and the glyoxylate bypass operon (aceBAK operon). It is known that the FadR protein represses the expression of the fad operon and the aceBAK operon when the concentration of fatty acid in a cell is high, while it increases the expression of the fatty acid biosynthesis-associated operon when the concentration of fatty acid in a cell is low (J. E. Cronan Jr. and D. Lapore, E. coli and Salmonella, vol 1, pp 211-214). The aceBAK operon is subject to biosynthesis of oxaloacetate from pyruvate which involves conversion of isocitrate, an intermediate of citric acid cycle (TCA cycle), into glyoxylate, of glyoxylate into malate, and of malate into oxaloacetate. Meanwhile, the citric acid cycle is subject to the biosynthesis of oxaloacetate from pyruvate through eight (8) pathways and, during the process, intermediates are converted into other metabolic intermediates including carbon dioxide. In this context, if the expression of the aceBAK operon is decreased, then the glyoxylate cycle is inhibited to increase the conversion rate of a carbon source into other metabolic intermediates through the citric acid cycle, which results in decreased production of L-threonine against identical amounts of carbon source.

Therefore, to develop a strain with the ability to produce a high concentration of L-threonine by increasing the aceBAK expression, it is necessary to knock out the *fadR* gene.

In addition, where an antibiotic marker is integrated into a chromosome, the marker can not be used again to knock out another gene. So the marker must be removed from chromosome, and the present inventors have applied the cre/loxp, site-specific recombination system to knock out the *fadR* gene.

The invention will be described further with reference to the accompanying drawings, in which:
FIG 1 depicts the construction of recombinant plasmid pT7Blue/fadR;
FIG 2 depicts the construction of DNA fragment ΔfadR::loxpcat from recombinant plasmid pT7fadR::loxpcat; and
FIG. 3 shows a Southern blot analysis of chromosomal recombination of the *fadR* gene.

In accordance with certain features of the present invention, the *E.coli* used for fermentation is characterized by the knockout of the *fadR* gene, which produces a regulatory factor for expression of the fad operon and the aceBAK operon. In certain embodiments, the present invention utilizes a mutated *E.coli* for L-threonine production by fermentation, in which the *fadR* gene present on the chromosome of the *E.coli* has been knocked out by homologous recombination such that the expression of the aceBAK operon is not repressed by the fadR protein and proceeds favourably during L-threonine biosynthesis.

In a preferred embodiment of the present invention, the *E.coli* is an L-threonine-producing strain of *Escherichia coli* which is resistant to L-methionine, L-threonine and L-lysine analogues and α-aminobutyric acid, and has a nutritional requirement for methionine and a leaky requirement for isoleucine. In another preferred embodiment of the present invention, the *E.coli* has, in addition to endogenous genes coding for phosphoenol pyruvate carboxylase (*ppc*) and enzymes included in the threonine operon, at least one exogenous copy of one or more of the *ppc* gene, and the thrA, thrB and thrC genes included in the threonine operon have been additionally introduced, preferably into the chromosomal DNA.

A knockout of the *fadR* gene present on the chromosome of a L-threonine-producing microorganism can be accomplished by an appropriate knockout mutation technique known to those skilled in the art. "Knockout mutation" as used herein refers to an *in vitro* engineered disruption of native, preferably chromosomal, DNA to prevent its biological function. Such disruption is typically within a protein coding region, and a foreign piece of DNA, conveniently but not necessarily providing a dominant selectable marker, may be inserted within the native sequence. A knockout mutation within a protein coding region prevents expression of the wild-type protein, which usually leads to loss of the function provided by the protein.

The knockout procedure can be carried out by mixing a knockout cassette with a culture of an L-threonine-producing microorganism competent for DNA uptake. While the microorganism is naturally transformable, it is preferred that cells can be rendered competent for DNA uptake by any suitable method (See e.g. LeBlanc et.al. Plasmid 28, 130-145, 1992; Pozzi et al. J. Bacteriol. 178, 6087-6090, 1996). A "knockout cassette" refers to a fragment of native, preferably chromosomal, DNA having a foreign DNA piece that may provide a selectable marker. In one embodiment "knockout mutation cassettes" are created by interrupting a fragment of genomic DNA with a foreign piece of DNA, and replacing the wild-type chromosomal copy of the sequence with the knockout cassette. In this embodiment, the knockout protocol involves cloning a foreign DNA piece into a target DNA such that "tails" comprising the target site DNA remain at the 5' and 3' ends of the knockout cassette. The tails may be at least 50 base pairs and preferably greater than 200 to 500 base pairs for efficient recombination and/or gene conversion. For convenience, the foreign DNA cloned into the target DNA also provides a selectable marker, for example, an antibiotic resistance gene. Where the target DNA is disrupted with an antibiotic resistance gene, selection of transformants may be carried out on agar plates containing suitable levels of an appropriate antibiotic. Following transformation, a portion of the cells that have taken up the knockout cassette will have undergone homologous recombination or gene conversion across the genomic DNA tails of the cassette, resulting in replacement of the wild-type genomic sequence by the knockout cassette. Knockout recombination events are easily confirmed by, for example, Southern blot hybridization, or more conveniently by PCR.

In one embodiment, a knockout mutation of the present invention comprises the following procedures. Genomic DNA is isolated from a strain that is capable of producing L-threonine and PCR is performed according to the conventional techniques using the genomic DNA as a template to amplify the *fadR* gene. The PCR product thus obtained, the *fadR* gene, is cloned into a suitable plasmid or other vectors and the resulting recombinant vector is introduced by transduction into a host cell such as *E. coli.* After the transformant is grown to proliferate in the culture medium, the recombinant vector having the *fadR* gene is extracted. An antibiotic resistance gene fragment is inserted within the *fadR* gene of the extracted recombinant vector to form a knockout recombinant vector. The knockout recombinant vector is introduced by transformation into a host cell and the host cell is cultivated in a suitable culture medium. Then, the propagated recombinant vector is isolated from the resultant transformant, and a knockout cassette fragment of the *fadR* gene is obtained by restriction enzyme treatment. Thereafter, this fragment is introduced into a host that is capable of producing L-threonine by a conventional technique such as electroporation.
As result, the wild-type *fadR* gene on the chromosome of the host is replaced with a *fadR* gene fragment comprising the antibiotic marker, and the host having a knocked-out *fadR* gene during repeated growth is isolated using antibiotic resistance

Skilled artisans will recognize that the knockout cassettes and the DNA segments of this invention or fragments thereof can be generated by general cloning methods. PCR amplification methods using oligonucleotide primers targeted to any suitable region of any of the sequences disclosed herein are preferred. Methods for PCR amplification are widely known in the art. See e.g. PCR Protocols: A Guide to Method and Application, Ed. M. Innis et al., Academic Press (1990) or U.S. Pat. No. 4,889,818. The PCR comprises genomic DNA, suitable enzymes, primers, and buffers, and is conveniently carried out in a DNA Thermal Cycler (Perkin Elmer Cetus, Norwalk, Conn. USA). A positive PCR result is determined by, for example, detecting an appropriately-sized DNA fragment following agarose gel electrophoresis.

The L-threonine-producing microorganism may be an L-threonine-producing strain of *Escherichia coli* which is resistant to L-methionine, L-threonine and L-lysine analogues and α-aminobutyric acid and have a nutritional requirement for methionine and a leaky requirement for isoleucine.

L-methionine analogues include, but are not limited to, D,L-ethionine, norleucine, α-methylmethionine and L-methionine-D,L-sulfoximine. L-threonine analogues include, but are not limited to, α-amino-β-hydroxy valeric acid and D,L-threonine hydroxamate. L-lysine analogues include, but are not limited to, S-(2-aminoethyl)-L-cysteine and δ-methyl-L-lysine.

In an especially preferred embodiment, the present invention uses, as the L-threonine-producing microorganism, a novel mutant of *Escherichia coli* KCCM 10236 which has the *fadR* gene knocked out on its chromosome and is capable of producing higher L-threonine than the parent strain KCCM 10236. This mutant was produced by transforming a knockout cassette ΔfadR::loxpcat into *Escherichia coli* KCCM 10236, which is resistant to L-methionine, L-threonine and L-lysine analogues and α-aminobutyric acid, and has a nutritional requirement for methionine and a leaky requirement for isoleucine, by electroporation. The knockout cassette ΔfadR::loxpcat is cleaved from recombinant plasmid construct pT7fadR::loxpcat which is derived from recombinant plasmid construct pT7blue/fadR. The novel mutant was designated as *Escherichia* coli FTR1201 and was deposited at Korean Culture Center of Microorganisms under accession number KCCM-10422 on September 13, 2002 according to the Budapest Treaty.

*Escherichia coli* KCCM 10236 was mutated from *Escherichia coli* KCCM TF4076 (KFCC 10718) which requires methionine and has resistances to L-threonine analogues (for example, α-amino-β-hydroxy valeric acid), L-lysine analogues (for example, S-(2-aminoethyl-L-cysteine), L-isoleucine analogues (for example, α-aminobutyric acid), methionine analogues (for example, ethionine) and the like.

*Escherichia coli* KCCM TF4076 is described in Korean Patent Publication No. 92-8365. *Escherichia coli* KCCM 10236 differs from *Escherichia coli* KCCM TF4076 in that it possesses two Phosphoenolpyruvate carboxylase *(ppc)* genes and two L-threonine *(thr)* operons. The *ppc* gene and *thr* operon from the chromosomes of *Escherichia coli* KCCM TF4076 were amplified by the polymerase chain reaction and were additionally integrated into the chromosomes of *Escherichia coli* KCCM TF4076 to generate *Escherichia coli* KCCM 10236.

The *ppc* gene catalyzes the formation of oxaloacetate from phosphoenolpyruvate. As such, phosphoenolpyruvate is a precursor of oxaloacetate which is an intermediate for the threonine biosynthetic pathway. *Escherichia coli* KCCM 10236 is therefore characterized in that it can enhance the expression of the *ppc* gene and the three genes involved in threonine biosynthesis from aspartate, *thrA, thrB* and *thrC,* encoding aspartokinase I/homoserine dehydrogenase, homoserine kinase and threonine synthase, respectively, resulting in a increase of L-threonine production.

In one embodiment, genomic DNA is extracted from *Escherichia coli* KCCM 10236. PCR is performed using the genomic DNA as a template to amplify the *fadR* gene. The PCR products are loaded onto agarose gel and subjected to electrophoresis. The *fadR* gene or DNA fragment thereof thus isolated is cloned into an appropriate vector. A DNA fragment of a gene conferring resistance to an antibiotic is inserted within the *fadR* gene region of the constructed recombinant vector to induce knockout of the *fadR* gene.

The knocked-out *fadR* gene cassette including the DNA fragment for antibiotic resistance is isolated from the resulting recombinant vector. The isolated knocked-out *fadR* gene cassette is transformed into a L-threonine-producing microorganism such as *Escherichia coli* KCCM 10236 by conventional methods, such as electroporation, to produce a mutated strain with the ability to highly produce L-threonine.

As used in the preparation of the knocked-out *fadR* gene or fragments thereof, the term "vector" refers to a nucleic acid molecule capable of carrying the foreign *fadR* gene or DNA fragment thereof to which it has been linked and introducing the *fadR* gene or DNA fragment into host cells. Examples of the vector are plasmids, cosmids, viruses and bacteriophages originating from natural sources or recombinantly synthesized.

The production of L-threonine by using the mutated L-threonine-producing microorganism of the present invention, in which the *fadR* gene (which may be present on the chromosome) of the microorganism has been rendered knocked out, can be carried out by an ordinary method for culturing host cells such as bacteria and yeast. As the medium used for the production of L-threonine, any synthetic media and natural media can be employed as long as it contains appropriate carbon sources, nitrogen sources, inorganic substances and trace amounts of nutrients which the strain requires.

Examples of the carbon sources include carbohydrates such as glucose, fructose, lactose, molasses, cellulose hydrolyzate, crude sugar hydrolyzate and starch hydrolyzate, organic acids such as pyruvic acid, acetic acid, fumaric acid, malic acid and lactic acid, and alcohols such as glycerin and ethanol. Examples of the nitrogen sources include ammonia, various inorganic salts (such as ammonium chloride, ammonium sulphate, ammonium acetate and ammonium phosphate), ammonium salts of organic acids, amines, peptone, meat extract, corn steep liquor, casein hydrolyzate, soybean cake hydrolyzate, various fermented cells and digested matters thereof.

Examples of the inorganic substances include potassium dihydrogenphosphate, dipotassium hydrogenphosphate, magnesium phosphate, magnesium sulphate, magnesium chloride, sodium chloride, ferrous sulphate, manganese sulphate, copper sulphate, calcium chloride and calcium carbonate.

Culturing is preferably carried out under aerobic conditions, for example, by shaking culture or spinner culture under aeration. The culturing temperature may be in the range of 20 to 40°C, preferably 28 to 37°C . The pH of the medium may be in the range of pH 5 to 9, preferably around neutrality. The pH adjustment may be carried out by using calcium carbonate, an organic or inorganic acid, an alkali solution, ammonia, a pH buffer, etc.

Usually, L-threonine is formed and accumulated in the culture by 1 to 7 days of culturing. An optional process for cultivation includes, for example, any of continuous operation, semi-continuous operation and batch operation.

After the culturing is completed, precipitates such as cells may be removed from the culture, and L-threonine can be recovered from the culture by means of ion exchange chromatography, concentration, salting-out, etc. alone or in combination. For example, the recovery of L-threonine from the culture broth can be carried out by the following method. The culture broth from which the cells are removed is adjusted to pH 2 with hydrochloric acid. Then the broth solution is passed through a strongly acidic ion exchange resin, and the adsorbant is eluted by dilute aqueous ammonia. Ammonia is evaporated and then the resulting solution is condensed. Alcohol is added to the concentrate, and then, crystals formed under cooling are collected, so that L-threonine can be obtained.

Preferred features of each aspect of the invention are as for each of the other aspects *mutatis mutandis.* The prior art documents mentioned herein are incorporated by reference to the fullest extent permitted by law.

The present invention will now be explained with reference to the following specific examples, which, however are not to be considered as limiting.

### Example 1 - Construction of Recombinant Plasmid and Knockout of fadR Gene

Genomic DNA was extracted from threonine-producing *Escherichia coli* strain KCCM 10236 by using the QIAGEN Genomic-tip System. A DNA fragment (about 0.8 kb) including the ORF (open reading frame) of the *fadR* gene was amplified by PCR using the extracted genomic DNA as a template and a pair of oligonucleotides 5'-TCG CGG AAG AGT ACA TTA TTG-3' (forward primer) and 5'-ATC GGC GCA AAG AAG TCC-3' (reverse primer). PCR was performed for 30 cycles, each consisting of denaturation for 30 seconds at 94°C, annealing for 30 seconds at 55°C and extension for 60 seconds at 72°C in that order.

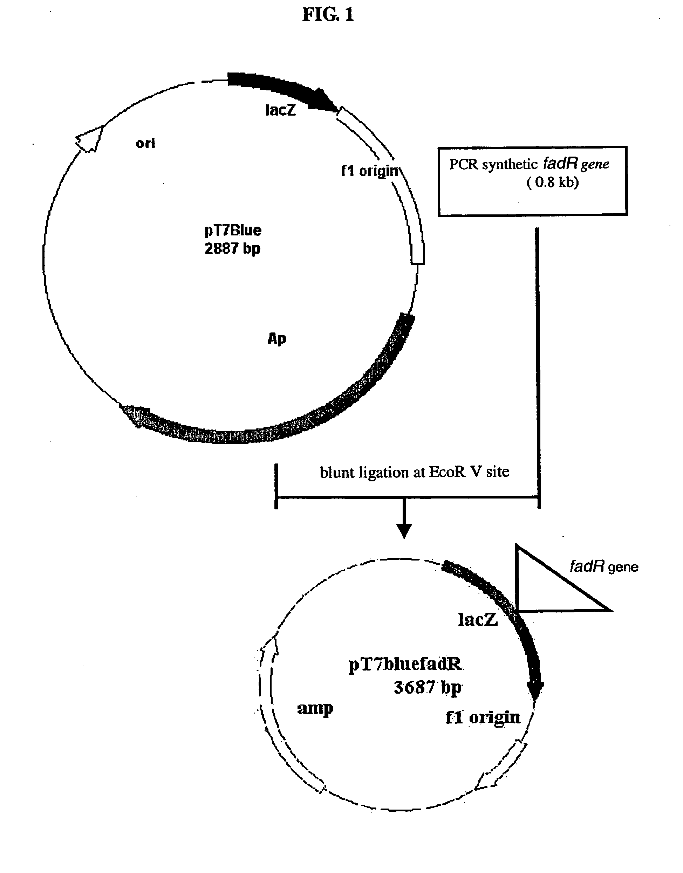

The PCR product was loaded onto 1.0% agarose gel and subjected to electrophoresis. The 0.8 kb *fadR* gene band was cut out of the gel and eluted. The resulting *fadR* gene was ligated to the EcoRV site of cloning vector pT7Blue (Novagen Inc., USA) overnight at a temperature of 16°C to construct the recombinant plasmid pT7Blue/fadR (see Fig. 1). The resulting plasmid construct was transformed into *Escherichia coli* NM522. The transformed strain was plated on solid media containing 50 mg/L of carbenicillin and was cultured overnight at a temperature of 37°C.

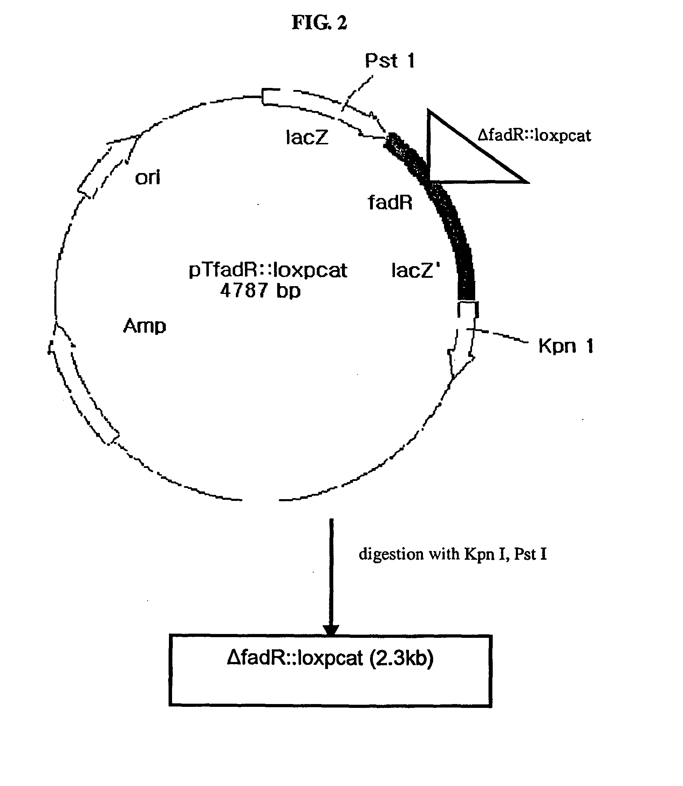

The formed colonies were picked up with a platinum loop and inoculated into 3 ml of liquid media containing carbenicillin. After overnight culturing, plasmid DNAs were extracted from the culture using QIAGEN Mini Prep Kit. The plasmid DNA extract was digested with the restriction enzyme SacII and confirmed the cloning of the *fadR* gene. The confirmed plasmid pT7Blue/fadR was cleaved with SacII and DNA fragments thus formed were run over a 0.8% agarose gel. The band at about 3.8 kb was cut out of the gel and eluted. The 3.8 kb DNA fragment was blunt-ended by the treatment of Klenow enzyme. The resulting DNA fragment was blunt-end ligated with about a 1.1 kb fragment of the gene for chloramphenicol resistance including loxp region, which was obtained by digesting plasmid ploxpcat2 (Gosset *et al,* A family of removable cassettes designed to obtain Ab-resistance-free genomic modifications of E. coli, Gene 247, pp255-264) with HincII restriction enzyme, to construct the approximately 5.0 kb recombinant plasmid pT7ΔfadR::loxpcat (see Fig. 2).

*Escherichia coli* NM522 was transformed with the recombinant plasmid pT7ΔfadR::loxpcat. The resulting transformant was streaked out onto a solid medium plate containing 50 mg/L of carbenicillin and 15 mg/L of chloramphenicol and cultured overnight at 32°C. The formed colonies were picked up with a platinum loop and inoculated into 3 ml of liquid media containing carbenicillin and chloramphenicol.

After overnight culturing, plasmid DNAs were extracted using QIAGEN Mini Prep Kit. The plasmid DNA extract was digested with KpnI and PstI restriction enzymes and DNA fragments formed thus were run over 0.7% agarose gel. The band at about 2.2 kb band was cut out of the gel and eluted (see Fig. 2). The resulting DNA fragment of about 2.2 kb, ΔfadR::loxpcat, was transformed into *Escherichia coli* KCCM 10236 by electroporation and the transformed *Escherichia coli* KCCM 10236 were streaked out onto an agar medium containing chloramphenicol.

### Example 2 - L-threonine Production in Erlenmeyer flask by Selected Strains

Thirty colonies selected in Example 1 were cultured in an Erlenmeyer flask to which the threonine titration medium given in Table 1 below was loaded, and L-threonine production was compared.

**TABLE 1**

| **Ingredients** | **Concentration (per liter)** |
|---|---|
| Glucose | 70 g |
| Ammonium sulfate | 28 g |
| KH₂PO₄ | 1.0 g |
| MgSO₄.7H₂O | 0.5 g |
| FeSO₄.7H₂O | 5 mg |
| MnSO₄.8H₂O | 5 mg |
| Calcium carbonate | 30 g |
| L-methionine | 0.15 g |
| Yeast extract | 2 g |
| pH 7.0 | |

Each colony was cultured on LB agar medium in a 32°C shaking incubator.

One platinum loop of the culture was inoculated into 25 ml of the titration medium and shaking cultured at 32°C and 250 rpm for 48 hours. The titration results are given in Table 2 below.

**TABLE 2**

| L-threonine | 23 g/L | 24 g/L | 25 g/L | 26 g/L |
|---|---|---|---|---|
| Strain number | 1 | 23 | 5 | 1 |

It can be seen from the results that the transformants of the present invention produce about 24-25 g/L L-threonine. Therefore, transformed *Escherichia coli* KCCM 10236 of the present invention in which the *fadR* gene has been knocked out is improved over the prototype strain KCCM 10236 which produces 23 g/L L-threonine. In addition, it was observed that the present transformed microorganisms increase the output of L-threonine in fermentation media by up to about 8% in comparison to the prototype strain. One transformant was designated as *Escherichia coli* FTR1201 (KCCM-10422).

### Example 3 - Confirmation of fadR Gene Knockout by Southern Blotting

A Southern blot was performed to confirm whether the *fadR* gene has been rendered knocked out in the strains selected in Example 2. The prototype strain KCCM 10236 and the present strains FTR1201 were cultured overnight on 3 ml of a liquid medium containing chloramphenicol. Genomic DNA was extracted from the culture using QIAGEN Genomic Kit 20.

The extracted genomic DNA was cleaved overnight with theXmnI restriction enzyme. Electrophoresis was conducted over 0.7% agarose gel to separate the resulting DNA fragments in size. After completion of electrophoresis, the DNA molecules in the agarose gel were transferred onto a nylon membrane (YOUNG Sci. Biodyne B Membrane) using a capillary transfer method (Molecular Cloning vol. 1. pp6.31-6.38). The membrane was dried and then the DNA molecules were immobilized on the dry membrane by UV irradiation (120 mJ/cm², SpectroLinker™).

The membrane was treated with a prehybridization solution I (Roche #1093657) at 55°C for 2 hours. After addition of the denatured DNA probe, hybridization was performed overnight in an oven at 55°C (BAMBINO 230300).

The denatured DNA probe used was prepared as follows. The isolated plasmid ploxpcat2 was digested with HincII restriction enzyme using QIAGEN Kit to obtain an approximately 1.1 kb DNA fragment of the gene for chloramphenicol resistance including the loxp region. The resulting DNA fragment was heated in water at 100°C for 5 minutes and soon cooled on ice for 5 minutes to give single-stranded DNA molecules which were DIG- labeled at 37°C using a DIG labeling and detection kit (Roche #1093657) to produce the DIG-UDP-labeled DNA probe.

After completion of the hybridization, the DNA molecules nonspecifically hybridized on the membrane were removed using the wash solutions I and II (Roche #1093657). The membrane was masked with the prehybridization buffer solution 2 (Roche #1093657) at a room temperature for 30 minutes and reacted with anti-DIG antibody, which specifically binds to DIG-UTP, at a room temperature for 30 minutes.

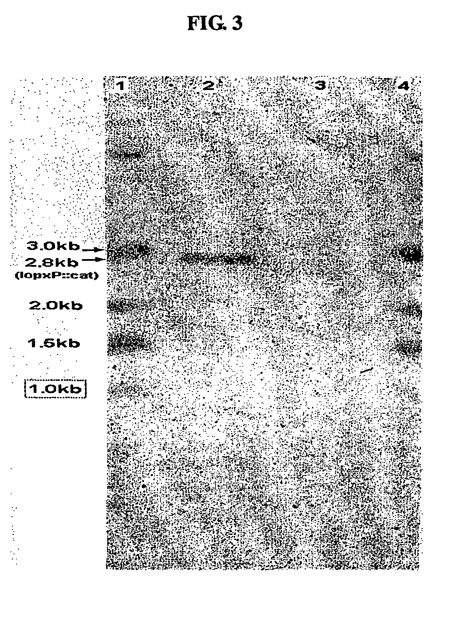

The anti-DIG antibody nonspecifically bound on the membrane was removed with the wash solution III (Roche #1093657) and the membrane was chromatized with the above label and detection kit (Roche #1093657) so as to show the band. The images were scanned using FLA-5000 Image System (FUJIFLIM). The results are given in Fig. 3. The prototype strain KCCM 10236 (lane 3) showed no band because it did not possess the gene for chloramphenicol resistance. By contrast, the strain FTR1201 (KCCM 10422) (lane 2) showed a band at about 2.8 kb as expected. It is understood that the band consists of a 1.7 kb portion of the *fadR* gene and an approximately 1.1 kb DNA fragment of the gene for chloramphenicol resistance.

### Example 4 - Production of L-threonine in a Fermenter

The production of L-threonine by the strain FTR1201 (KCCM-10422) in a 5 L fermenter was compared with that by the prototype strain KCCM 10236. The contents of the inoculum medium used are shown in Table 3 below.

**TABLE 3**

| **Ingredients** | **Concentration (per liter)** |
|---|---|
| Glucose | 50 g |
| KH₂PO₄ | 4 g |
| (NH₄)₂SO₄ | 6 g |
| Yeast extract | 3 g |
| MgSO₄·7H₂O | 2 g |
| L-methionine | 1 g |
| FeSO₄·7H₂O | 40 mg |
| MnSO₄·8H₂O | 10 mg |
| CaCl₂·2H₂O | 40 mg |
| CoCl₂·6H₂O | 4 mg |
| H₃BO₃ | 5 mg |
| Na₂MoO₄·2H₂O | 2 mg |
| ZnSO₄·7H₂O | 2 mg |
| pH 7.0 | |

A LB medium supplemented with 10 g/L of glucose and 0.1 g/L of L-methionine was used for the seed culture. The initial inoculum volume in the fermenter was adjusted to from 3% to 5% of the initial culture volume. Glucose was added six times each at its depletion point. After each addition, the concentration of glucose was 5%. At the time of adding glucose, 1% by weight of mono-potassium phosphate (KH₂PO₄) was also added. The initial and final culture volumes were 1.5 L and 3.0 L, respectively, and the concentration of total glucose added until completion of fermentation was 250 g/L. Fermentation was performed with aeration of 0.5 vvm and stirring of 1,000 rpm at a temperature of 32°C. The pH was maintained at 7.0 by an automatic inlet providing from 25% to 28% of ammonium gas.
The results are given in Table 4 below.

**TABLE 4**

| **Strain** | **L-threonine Conc. (g/L)** | **Yield (%)** |
|---|---|---|
| Prototype KCCM 10236 | 93.5 | 37.4 |
| FTR1201 KCCM 10422 | 102 | 41 |

The prototype strain 10236 produced 93.5 g/L of L-threonine at 37.4% yield to the glucose consumed. As contrast, the strain FTR1201 produced 102 g/L of L-threonine at 41 % yield to the glucose consumed and thus increased about 9 % of L-threonine yield in comparison to the prototype strain.

The L-threonine-producing mutated microorganism of the present invention in which the *fadR* gene has been knocked out enhances the production of L-threonine.

## Claims

1. An L-threonine-producing *E. coli* which is resistant to α-aminobutyric acid, D,L-ethionine, α-amino-β-hydroxy valeric acid, and S-(2-aminoethyl)-L-cystein and in which the *fadR* gene has been knocked out.

2. An *E. coli* as claimed in claim 1, the DNA of which contains at least one additional copy of the *ppc* gene, the *thrA* gene, the *thrB* gene, the *thrC* gene or a combination thereof.

3. An *E. coli* as claimed in claim 1 or claim 2, which is *Escherichia coli* FTR 1201 (KCCM-10422).

4. A method for producing an *E. coli* as claimed in claim 1, which comprises (a) introducing a knockout cassette of the *fadR* gene into an L-threonine-producing *E. coli* which is resistant to α-aminobutyric acid, D,L-ethionine, α-amino-β-hydroxy valeric acid, and S-(2-aminoethyl)-L-cystein, to cause homologous recombination to occur between the cassette and the *fadR* gene of the *E. coli,* and (b) selecting a mutated *E. coli* in which the *fadR* gene has been rendered knocked-out.

5. The method of claim 6, wherein the knockout cassette of the *fadR* gene comprises an antibiotic marker inserted within the *fadR* gene such that it is knocked out.

6. A method for producing L-threonine which comprises culturing the *E. coli* of any one of claims 1 to 3 and isolating the L-threonine from the culture.

## Patentansprüche

1. L-threoninproduzierende E. coli, die gegenüber α-Aminobuttersäure, D,L-Ethionin, α-Amino-β-Hydroxyvaleriansäure und S-(2-Aminoethyl)-L-cystein resistent ist, und bei dem das *fadR*-Gen ausgeknockt wurde.

2. *E. coli* nach Anspruch 1, deren DNA zumindest eine zusätzliche Kopie des *ppc*-Gens, des thrA-Gens, des thrB-Gens, des *thrC-*Gens oder einer Kombination davon enthält.

3. *E. coli* nach Anspruch 1 oder 2, bei der es sich um Escherichia coli FTR 1201 (KCCM-10422) handelt.

4. Verfahren zum Herstellen einer *E. coli* nach Anspruch 1, welches (a) das Einbringen einer Knockout-Kassette des *fadR*-Gens in eine L-threoninproduzierende *E. coli,* die gegenüber α-Aminobuttersäure, D,L-Ethionin, α-Amino-β-Hydroxyvaleriansäure und S-(2-Aminoethyl)-L-cystein resistent ist, um eine homologe Rekombination zwischen der Kassette und dem *fadR*-Gen der *E. coli* zu verursachen, und (b) das Auswählen einer mutierten *E. coli,* bei der das *fadR*-Gen ausgeknockt wurde, umfasst.

5. Verfahren nach Anspruch 6, wobei die Knockout-Kassette des *fadR*-Gens einen antibiotischen Marker umfasst, der im *fadR*-Gen eingesetzt ist, so dass dieses ausgeknockt wird.

6. Verfahren zum Herstellen von L-Threonin, welches das Züchten der *E. coli* nach einem der Ansprüche 1 bis 3 und das Isolieren des L-Threonins aus der Kultur umfasst.

## Revendications

1. E. coli produisant de la L-thréonine qui est résistante à l'acide α-aminobutyrique, à la D,L-éthionine, à l'acide α-amino-β-hydroxy valérique et à la S-(2-aminoéthyl)-L-cystéine et dans laquelle le gène fadR a été désactivé.

2. E. coli selon la revendication 1, dont l'ADN contient au moins une copie supplémentaire du gène ppc, du gène thrA, du gène thrB, du gène thrC ou d'une combinaison de ceux-ci.

3. E coli selon la revendication 1 ou la revendication 2, laquelle est Escherichia coli FTR 1201 (KCCM-10422).

4. Procédé de production d'une E. coli selon la revendication 1, comprenant (a) l'introduction d'une cassette désactivatrice du gène fadR dans une E. coli produisant de la L-thréonine qui est résistante à l'acide α-aminobutyrique, à la D,L-éthionine, à l'acide α-amino-β-hydroxy valérique et à la S-(2-aminoéthyl)-L-cystéine pour provoquer une recombinaison homologue entre la cassette et le gène fadR de E. coli, (b) la sélection d'une E. coli mutée dans laquelle le gène fadR a été désactivé.

5. Procédé selon la revendication 6, dans lequel la cassette désactivatrice du gène fadR comprend un marqueur antibiotique inséré dans le gène fadR de manière à le désactiver.

6. Procédé de production de L-thréonine, comprenant la culture de E. coli selon l'une quelconque des revendications 1 à 3 et l'isolement de la L-thréonine de la culture.
